# EUROPEAN PATENT APPLICATION

(11) **EP 2 116 534 A1**
(43) Date of publication of application: **11.11.2009**
(21) Application number: 08704197.6
(22) Date of filing: 30.01.2008
(51) Int. Cl.: C07D 213/79, C07D 215/50, C07D 233/54, C07D 235/04, C09B 57/10, H01L 51/42, H01M 14/00, C07F 15/00

(54) **PROCESS FOR PRODUCTION OF BINUCLEAR METAL COMPLEX**

(30) Priority: 31.01.2007 JP 2007022098
(71) Applicant: Ube Industries, Ltd., Ube-shi Yamaguchi 755-8633 (JP)
(72) Inventor: IWASA, Takafumi, Ichihara-shi Chiba 290-0045 (JP); KAKUTA, Yoshihisa, Ichihara-shi Chiba 290-0045 (JP)
(74) Representative: Cabinet Plasseraud
(86) International application number: PCT/JP2008/051434
(87) International publication number: WO 2008/093742

(57) **Abstract**

Disclosed is a process for production of an asymmetric binuclear metal complex represented by the general formula: (L¹)₂M¹(BL)M²(L²)₂(X)ₙ wherein M¹ and M², which may be the same as or different from each other, represent a transition metal; L¹ and L², which are different from each other, represent a chelate ligand capable of multidentate coordination and two L¹s may be different from each other and two L²s may be different from each other; BL represents a bridging ligand having at least two cyclic structures each containing a hetero atom, the hetero atoms contained in the cyclic structures being ligand atoms coordinating to M¹ and M²; X represents a counter ion; and n is the number of counter ions needed to neutralize the charge of the complex. In the process, the binuclear metal complex is isolated by adjusting the pH of the solution containing the binuclear metal complex to a value higher than 2.5. The binuclear metal complex obtained may be used as a dye to produce a photoelectric conversion element and a photochemical battery having higher photoelectric conversion efficiency and higher durability.

## Description

### Technical Field

The present invention relates to a novel process for production of a binuclear metal complex.

The present invention also relates to a metal complex dye produced by the process; a photoelectric conversion element comprising an oxide semiconductor photosensitized by the metal complex dye; and a photochemical battery therewith.

### Background Art

A solar battery is greatly expected to be a clean regenerative energy source, and researches have been conducted for practical application of a monocrystalline-silicon, polycrystalline-silicon or amorphous-silicon solar battery and a solar battery containing a compound such as cadmium telluride and indium-copper selenide. For prevalence of it as a household power source, however, any of these batteries faces many problems to be overcome, including a higher production cost, difficulty in ensuring raw material preparation, difficulty in recycling, and difficulty in realizing a larger area. Therefore, there have been proposed solar batteries wherein an organic material is used in an attempt to achieve a larger area and a lower cost. However, any of these has a conversion efficiency of about 1 %, which falls very short of practical use.

Under such circumstances, in 1991, Graetzel et al. disclosed a photoelectric conversion element comprising semiconductor particles sensitized by a dye and a solar battery, as well as materials and technique for producing the solar battery in Nature (for example, Nature, Vol. 353, p. 737, 1991 (Non-patent document 1) and JP-A-1989-220380 (Patent document 1)). The battery is a wet solar battery having a porous titania film sensitized by a ruthenium dye as a working electrode. This solar battery has the advantages that it can be provided as an inexpensive photoelectric conversion element because inexpensive materials can be used without highly purification, and that solar light can be converted into electricity over a wide visible light wavelength range because a dye having broad absorption is used. However, the conversion efficiency must be further improved for practical use, and therefore, it has been desired to develop a dye which has a higher absorbance index and absorb higher wavelength light.

JP-A-2003-261536 (Patent document 2) by the present applicant has disclosed a mononuclear metal complex containing a dipyridyl ligand, which is a metal complex dye useful as a photoelectric conversion element.

"Current Technology in Dye-sensitized Solar Battery" (CMC Co., LTD., published on May 25, 2001, p.117) (Non-patent document 2) has disclosed a polynuclear β-diketonate complex dye.

JP-A-2004-359677 (Patent document 3) has disclosed a polynuclear complex comprising a plurality of metals and a plurality of ligands wherein a bridging ligand (BL) coordinating to the plurality of metals has a coordination structure having a conjugated heterocyclic ring and a coordination structure without a conjugated heterocyclic ring, as a novel polynuclear complex improved in photoelectric conversion function of emitting electrons while receiving energy from active ray such as light.

In addition, WO 2006/038587 A1 (Patent document 4) has disclosed a binuclear metal complex having a coordination structure with a conjugated heterocyclic ring, as a metal complex dye which allows for the higher photoelectric conversion efficiency of photoelectric conversion elements. In Examples of Patent document 4, the binuclear metal complex was isolated by adding an acid to the reaction solution to adjust the pH of the solution to 2.5 after the completion of the synthesis reaction.

As a dye used for a photoelectric conversion element, there has been needed a metal complex dye for the production of photoelectric conversion elements having a higher photoelectric conversion efficiency and excellent durability.

### List of References

Patent document 1: JP-A-1989-220380;
Patent document 2: JP-A-2003-261536;
Patent document 3: JP-A-2004-359677;
Patent document 4: WO 2006/038587 A1;
Non-patent document 1: Nature, Vol. 353, p. 737, 1991;
Non-patent document 2: "Current Technology in Dye-sensitized Solar Battery" (CMC Co., LTD., published on May 25, 2001, p.117).

### Disclosure of the Invention

### Problems to be Solved by the Invention

An objective of the present invention is to provide a process for producing a binuclear metal complex which is useful as a metal complex dye and allows the higher photoelectric conversion efficiency and higher durability of photoelectric conversion elements and photochemical batteries.

### Means for Soling the Problems

The present invention relates to the followings.

[1] A process for production of an asymmetric binuclear metal complex, comprising a step of:
   isolating the binuclear metal complex by adjusting the pH of the solution containing the binuclear metal complex to a value higher than 2.5; wherein
   the binuclear metal complex is a complex represented by the general formula: (L¹)₂M¹(BL)M²(L²)₂(X)ₙ
      in which
   M¹ and M², which may be the same as or different from each other, represent a transition metal;
   L¹ and L², which are different from each other, represent a chelate ligand capable of multidentate coordination, and two L¹s may be different from each other, and two L²s may be different from each other;
   BL represents a bridging ligand having at least two cyclic structures each containing a hetero atom, the hetero atoms contained in the cyclic structures being ligand atoms coordinating to M¹ and M²;
   X represents a counter ion; and
   n is the number of counter ions needed to neutralize the charge of the complex.

[2] The production process as described in [1], wherein the binuclear metal complex represented by the formula: (L¹)₂M²(BL)M²(L²)₂(X)ₙ is isolated by adjusting the pH of the solution to 2.7 to 5.

[3] The production process as described in [2], wherein the binuclear metal complex represented by the formula: (L¹)₂M¹(BL)M²(L²)₂(X)ₙ is isolated by adjusting the pH of the solution to 3.3 to 5.

[4] The production process as described in any one of [1] to [3], wherein the solution is a reaction solution obtained by reacting a mononuclear metal complex represented by the formula: (L¹)₂M¹Cl₂ (wherein M¹ and L¹ have the meanings indicated above) with a mononuclear metal complex represented by the formula: (BL)M²(L²)₂ (wherein M², L² and BL have the meanings indicated above) in the presence of a base in a solvent, or a solution obtained by dissolving a binuclear metal complex represented by the formula: (L¹)₂M¹(BL)M²(L²)₂(X)ₙ in water.

[5] An asymmetric binuclear metal complex produced by the process as described in any one of [1] to [4], and represented by the general formula: (L¹)₂M¹(BL)M²(L²)₂(X)ₙ
   in which
   M¹ and M², which may be the same as or different from each other, represent a transition metal;
   L¹ and L², which are different from each other, represent a chelate ligand capable of multidentate coordination, and two L¹s may be different from each other, and two L²s may be different from each other;
   BL represents a bridging ligand having at least two cyclic structures each containing a hetero atom, the hetero atoms contained in the cyclic structures being ligand atoms coordinating to M¹ and M²;
   X represents a counter ion; and
   n is the number of counter ions needed to neutralize the charge of the complex.

[6] A metal complex dye comprising an asymmetric binuclear metal complex produced by the process as described in any one of [1] to [4], and represented by the general formula: (L¹)₂M¹(BL)M²(L²)₂(X)ₙ in which
   M¹ and M², which may be the same as or different from each other, represent a transition metal;
   L¹ and L², which are different from each other, represent a chelate ligand capable of multidentate coordination, and two L¹s may be different from each other, and two L²s may be different from each other;
   X represents a counter ion;
   n is the number of counter ions needed to neutralize the charge of the complex;
   BL represents a bridging ligand having at least two cyclic structures each containing a hetero atom, the hetero atoms contained in the cyclic structures being ligand atoms coordinating to M¹ and M²; and
   L¹ contains a substituent capable of attaching to a semiconductor particle; and
   LUMOs are predominantly distributed in (L¹)₂M¹.

[7] A photoelectric conversion element comprising semiconductor particles sensitized by the metal complex dye as described in [6].

[8] The photoelectric conversion element as described in [7], wherein the semiconductor particle is selected from the group consisting of titanium oxide, zinc oxide and tin oxide.

[9] A photochemical battery comprising the photoelectric conversion element as described in any one of [7] to [8].

### Effect of the Invention

According to the present invention, the binuclear metal complex represented by the formula: (L¹)₂M¹(BL)M²(L²)₂(X)ₙ is isolated by adjusting the pH of the solution containing the binuclear metal complex to a value higher than 2.5, preferably 2.7 to 5. When using a binuclear metal complex dye obtained according to the present invention, the photochemical battery obtained may have a higher initial photoelectric conversion efficiency, as compared to a conventional binuclear metal complex dye which is isolated at pH 2.5. Furthermore, durability of the photochemical battery obtained may be improved when using a binuclear metal complex which is isolated at a pH of 3.3 to 5, as compared to when using a binuclear metal complex which is isolated at a lower pH.

### Brief Description of the Drawings

FIG. 1 is a graph showing the photoelectric conversion efficiency of the photochemical batteries which comprise the binuclear metal complex dyes prepared in Examples 2 to 4 and Comparative Example 1, respectively.
FIG. 2 is a graph showing the photoelectric conversion efficiency of the photochemical batteries which comprise the binuclear metal complex dyes prepared in Examples 1 and 3, respectively, after being left in the dark at 85 °C for a predetermined time.

### Best Mode for Carrying Out the Invention

According to the present invention, the asymmetric binuclear metal complex represented by the general formula: (L¹)₂M¹(BL)M²(L²)₂(X)ₙ may be prepared by reacting two mononuclear metal complexes (L¹)₂M¹Cl₂ and (BL)M²(L²)₂ which are synthesized as described below, for example.

A mononuclear metal complex (L¹)₂M¹Cl₂ (M¹C-1) in which L¹ is a ligand represented by the formula (L¹-1):

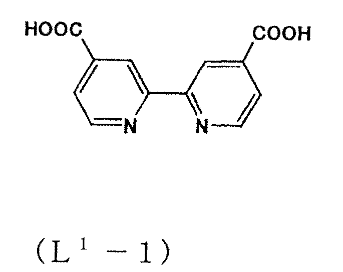

and M¹ is Ru may be prepared as shown in the following synthetic scheme.

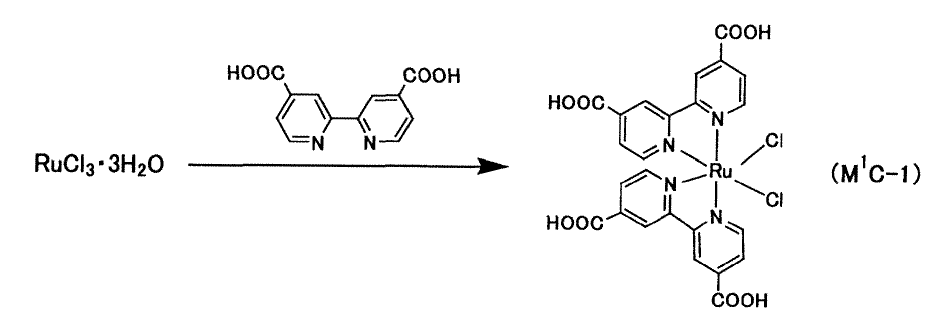

In the above synthetic scheme, a complex in which L¹ has a substituent other than carboxyl, and a complex in which M¹ is a transition metal other than Ru may be synthesized in a similar way.

A mononuclear metal complex (L¹)₂M¹Cl₂ (M¹C-2) in which L¹ is a ligand represented by the formula (L¹-4):

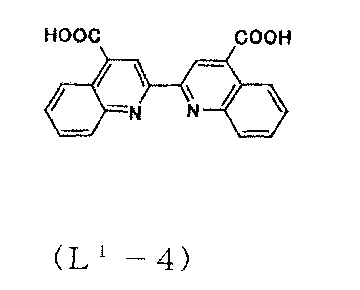

and M¹ is Ru may be prepared as shown in the following synthetic scheme.

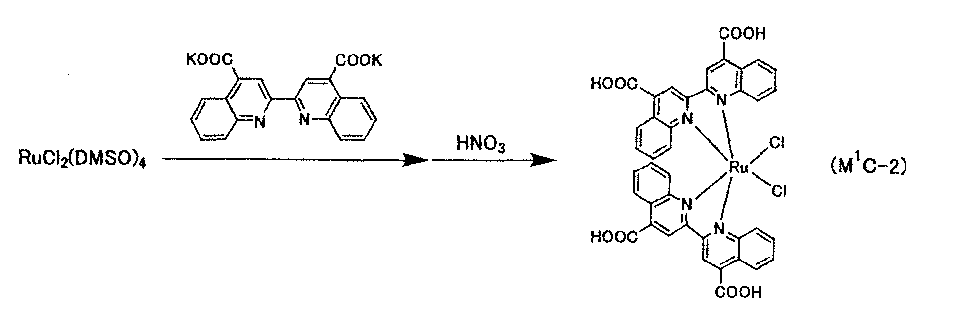

In the above synthetic scheme, a complex in which L¹ has a substituent other than carboxyl, and a complex in which M¹ is a transition metal other than Ru may be synthesized in a similar way.

On the other hand, a mononuclear metal complex (BL)M²(L²)₂ may be prepared as shown in the following synthetic scheme.

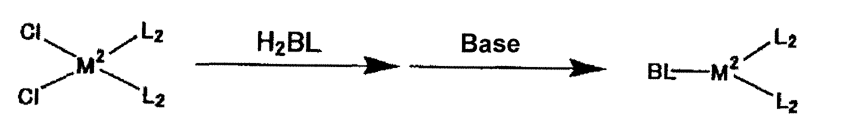

In the above synthetic scheme, H₂BL represents a compound in which two hetero atoms (nitrogen, and so on) in BL are protonated.

Any of complexes in which BL is a ligand represented by any of the formulas (BL-1) to (BL-4) (including those having a substituent) described below, and any of complexes in which L² is a ligand represented by any of the formulas (L²-1) to (L²-4) (including those having a substituent) described below may be synthesized as shown in this synthetic scheme. For a complex in which BL is a ligand represented by the formula (BL-1) (including that having a substituent), the latter reaction step using a base may be omitted, and M²(L²)₂Cl₂ and BL may be reacted to give (BL)M²(L²)₂.

A base used in the reaction may be preferably a base which does not contain sodium such as bases containing potassium, magnesium, calcium or iron, and organic bases, more preferably bases containing lithium. Among them, the base may be particularly preferably lithium alkoxide, more preferably lithium methoxide, lithium ethoxide, or lithium t-butoxide, particularly preferably lithium methoxide. The amount of the base to be used may be appropriately determined.

(L¹)₂M¹Cl₂ (M¹C) and (BL)M²(L²)₂ (M²C) thus synthesized may be reacted as shown in the following synthetic scheme to give (L¹)₂M¹(BL)M²(L²)₂(X)ₙ.

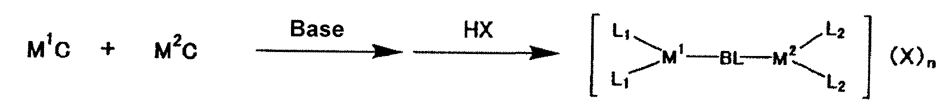

A base used in the reaction may be preferably a base which does not contain sodium such as bases containing potassium, magnesium, calcium or iron, and organic bases, more preferably bases containing lithium. Among them, the base may be particularly preferably lithium hydroxide, or lithium alkoxide, more preferably lithium hydroxide, lithium methoxide, lithium ethoxide, or lithium t-butoxide, particularly preferably lithium hydroxide.
The amount of the base to be used may be appropriately determined.

After reacting (L¹)₂M¹Cl₂ (M¹C) with (BL)M²(L²)₂ (M²C) in the presence of a base, an acid (HX) may be added to the reaction solution to isolate the binuclear metal complex represented by the formula: (L¹)₂M¹(BL)M²(L²)₂(X)ₙ. While adding an acid, the reaction solution may be cooled, if necessary. A lithium salt (LiX) such as lithium nitrate may be added to the reaction solution prior to or simultaneously with adding an acid.

According to the present invention, the pH of the reaction solution is adjusted to a value higher than 2.5, preferably 2.7 or higher to isolate the binuclear metal complex. The binuclear metal complex may be preferably isolated at a pH of 2.8 or higher. In view of durability of the photochemical battery obtained, the binuclear metal complex may be preferably isolated at a pH of 3.3 or higher, more preferably 3.5 or higher, particularly preferably 3.7 or higher, further preferably 3.8 or higher. In addition, the binuclear metal complex may be preferably isolated at a pH of 5 or lower, more preferably 4.5 or lower, particularly preferably 4.2 or lower for sufficiently depositing the binuclear metal complex dye of the present invention on semiconductor particles.

Alternatively, the binuclear metal complex represented by the formula: (L¹)₂M¹(BL)M²(L²)₂(X)ₙ may be isolated by suspending the binuclear metal complex represented by the formula: (L¹)₂M¹(BL)M²(L²)₂(X)ₙ, which is isolated at an appropriate pH, in water; adding a base to the suspension until the pH become about 10 to redissolve the binuclear metal complex in water; and then adding an acid to the solution to adjust the pH within the above range.

A metal complex according to the present invention may be prepared by reference to the known processes, for example, the process described in the literatures, WO 2006/038587, Inorganic Chemistry, Vol.17 (9), pp.2660-2666, 1978, and Journal of the American Chemical Society, Vol.115, pp.6382-6390, 1993, except that the pH is adjusted within the above range to isolate the binuclear metal complex.

The binuclear metal complex produced according to the present invention will now be described.

In the asymmetric binuclear metal complex represented by the general formula: (L¹)₂M¹(BL)M²(L²)₂(X)ₙ according to the present invention, M¹ and M² represent a transition metal, preferably a transition metal in Groups VIII to XI. Specifically, ruthenium (Ru), osmium (Os), cobalt (Co), nickel (Ni), copper (Cu) or iron (Fe) is preferable. Among them, ruthenium (Ru) and osmium (Os) are more preferable, and ruthenium (Ru) is particularly preferable.

M¹ and M² may be the same as or different from each other.

L¹ and L² represent a chelate ligand capable of multidentate coordination, preferably a chelate ligand capable of bidentate, tridentate or tetradentate coordination, more preferably a chelate ligand capable of bidentate coordination. Specific examples include derivatives of 2,2'-bipyridine, 1,10-phenanthroline, 2-(2-pyridinyl)quinoline, 2,2'-biquinoline and the like. L¹ and L² are different from each other. Two L¹s may be different from each other, and two L²s may be also different from each other.

When the binuclear metal complex according to the present invention is a metal complex dye used for a photoelectric conversion element, L¹ contains at least one substituent capable of attaching to a semiconductor particle.

Examples of a substituent in L¹ capable of attaching to a semiconductor particle include carboxyl (-COOH), amino (-NH₂), hydroxy (-OH), sulfate (-SO₃H), phosphate (-PO₃H₂) and nitro (-NO₂). Among them, carboxyl (-COOH) is preferable. The hydrogen in a carboxyl group may be replaced with a cation including a quaternary ammonium such as tetrabutylammonium and an alkali metal ion such as sodium ion. Alternatively, the hydrogen may be liberated from the complex. When the binuclear metal complex according to the present invention is isolated at a higher pH, the hydrogen in the carboxyl group tends to be liberated. In case of the binuclear metal complex which contains two L¹s each having two carboxyl groups, and therefore has four carboxyl groups in total, an isolate containing the binuclear metal complex in which one hydrogen in the carboxyl groups is liberated may be obtained by isolating at a pH of 2.7 to 3.0, and an isolate containing the binuclear metal complex in which two hydrogens in the carboxyl groups are liberated may be obtained by isolating at a pH of 3.5 to 4.0.

In addition, L¹ may or may not contain a substituent other than the substituent capable of attaching to a semiconductor particle. Examples of such a substituent include alkyl such as methyl and ethyl, and alkoxy such as methoxy and ethoxy.

When the binuclear metal complex according to the present invention is a metal complex dye used for a photoelectric conversion element, L¹ is preferably a ligand in which LUMOs are predominantly distributed in the (L¹)₂M¹ moiety. The phrase, "LUMOs are predominantly distributed in the (L¹)₂M¹ moiety" as used herein means that there are more LUMOs in the (L¹)₂M¹ moiety than in the (L²)₂M² moiety. The structure of the binuclear metal complex where the (L¹)₂M¹ predominantly have LUMOs to which an electron is excited by irradiation with light such as solar light allows smooth electron transfer from an electrolyte to a photoelectric conversion element (anode) when a photochemical battery is produced using a photoelectric conversion element comprising semiconductor particles sensitized by the binuclear metal complex. Consequently, an efficient photochemical battery may be obtained.

An LUMO was calculated using software, Cerius² or Material Studio. The method was optimized for a metal complex structure by DFT (density functional theory) using the DMol³ module. Suitable exchange correlation functions therefor include, but not limited to, VWN and BLYP methods. A suitable basis function is, but not limited to, DNP.

An energy state was calculated using the obtained structure, and an exchange correlation function therefor is, but not limited to, BLYP and PBE, and a suitable basis function is, but not limited to, DNP.

L¹ may be the ligand represented by the following formula (L¹-A).

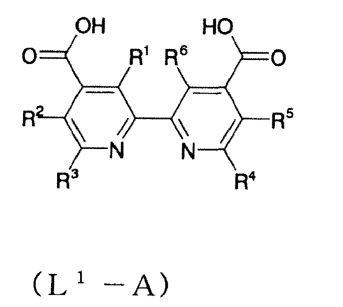

In the above formula, H in -COOH may be liberated. R¹, R², R³, R⁴, R⁵ and R⁶ independently represent hydrogen, alkoxy, or substituted or unsubstituted hydrocarbon, or alternatively, two or more of these together with the carbon atoms to which they are bound may form a substituted or unsubstituted aromatic hydrocarbon ring, or a substituted or unsubstituted aliphatic hydrocarbon ring.

R¹ to R⁶ are preferably hydrogen, alkyl or alkoxy, more preferably hydrogen or alkyl. The alkyl is preferably one containing up to six carbon atoms, more preferably methyl or ethyl. The alkoxy is preferably one containing up to six carbon atoms, more preferably methoxy or ethoxy.

It is also preferable that R² and R³, R⁴ and R⁵, or R¹ and R⁶ together with the carbon atoms to which they are bound form a six-membered aromatic hydrocarbon ring which may be substituted with a substituent. Examples of the substituent in the aromatic hydrocarbon ring include alkyl such as methyl and ethyl, and alkoxy such as methoxy and ethoxy.

R¹ to R⁶ are particularly preferably hydrogen.

Specific examples of L¹ include, but are not limited to, the ligands represented by the following formulas (L¹-1) to (L¹-4).

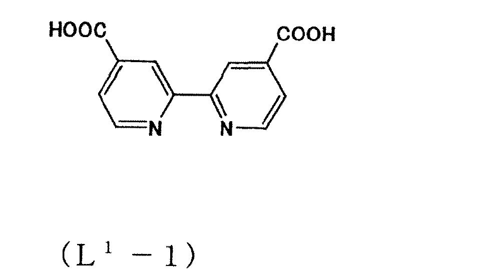

2,2'-Bipyridine-4,4'-dicarboxylic acid (H₂dcbpy)

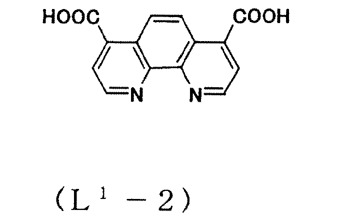

1,10-Phenanthroline-4,7-dicarboxylic acid (H₂dcphen)

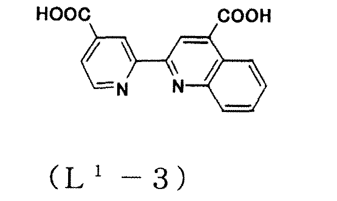

2-(2-(4-Carboxypyridyl))-4-carboxyquinoline (H₂dcpq)

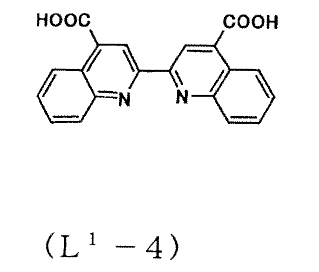

2,2'-Biquinoline-4,4'-dicarboxylic acid (H₂dcbiq)
In these formulas (L¹-1) to (L¹-4), the heterocyclic ring and the benzene ring may be substituted with a substituent, and H in -COOH may be liberated. Examples of the substituent include alkyl containing up to six carbon atoms such as methyl and ethyl, and alkoxy containing up to six carbon atoms such as methoxy and ethoxy.

As described above, L² is a chelate ligand capable of multidentate coordination, preferably a chelate ligand capable of bidentate, tridentate or tetradentate coordination, more preferably a chelate ligand capable of bidentate coordination. Specific examples include derivatives of 2,2'-bipyridine, 1,10-phenanthroline, 2-(2-pyridinyl)quinoline, 2,2'-biquinoline and the like.

L² may or may not contain a substituent. Examples of the substituent in L² include alkyl such as methyl and ethyl, aryl such as phenyl and tolyl, alkoxy such as methoxy and ethoxy, and hydroxy (-OH). An electron-donating group is particularly preferable.

L² may be the ligand represented by the following formula (L²-A).

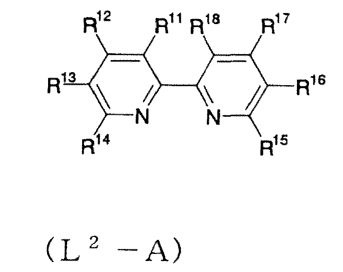

In the above formula, R¹¹_{.} R¹², R¹³, R¹⁴, R¹⁵, R¹⁶, R¹⁷ and R¹⁸ independently represent hydrogen, alkoxy, hydroxy, or substituted or unsubstituted hydrocarbon, or alternatively, two or more of these together with the carbon atoms to which they are bound may form a substituted or unsubstituted aromatic hydrocarbon ring, or a substituted or unsubstituted aliphatic hydrocarbon ring.

R¹¹ to R¹⁸ are preferably hydrogen, alkyl or alkoxy, more preferably hydrogen or alkyl. The alkyl is preferably one containing up to six carbon atoms, more preferably methyl or ethyl. The alkoxy is preferably one containing up to six carbon atoms, more preferably methoxy or ethoxy.

It is also preferable that adjacent two of R¹¹ to R¹⁸ or R¹¹ and R¹⁸ together with the carbon atoms to which they are bound form a six-membered aromatic hydrocarbon ring which may be substituted with a substituent. Examples of the substituent in the aromatic hydrocarbon ring include alkyl such as methyl and ethyl, and alkoxy such as methoxy and ethoxy.

R¹¹ to R¹⁸ are particularly preferably hydrogen or methyl. Alternatively, it is also particularly preferable that R¹¹ and R¹⁸ together with the carbon atoms to which they are bound form a six-membered aromatic hydrocarbon ring which may be substituted with a substituent such as methyl, and R¹² to R¹⁷ are hydrogen or methyl, more preferably hydrogen.

Specific examples of L² include, but are not limited to, the ligands represented by the following formulas (L²-1) to (L²-4).

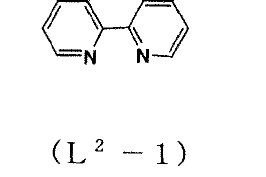

2,2'-Bipyridine (bpy)

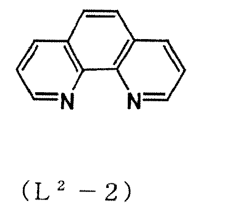

1,10-Phenanthroline (phen)

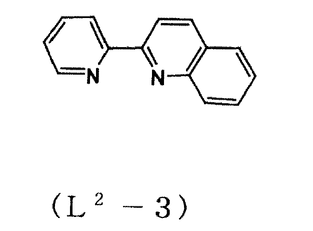

2-(2-Pyridinyl)quinoline (pq)

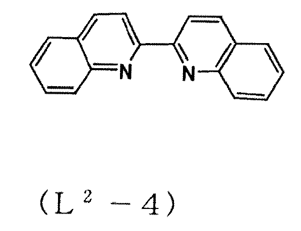

2,2'-Biquinoline (biq)
In these formulas (L²-1) to (L²-4), the heterocyclic ring and the benzene ring may be substituted with a substituent. Examples of the substituent include alkyl containing up to six carbon atoms, alkoxy containing up to six carbon atoms, phenyl optionally substituted with a substituent such as methyl, and hydroxy.

BL is a bridging ligand and has a cyclic structure containing a hetero atom. The hetero atom contained in the cyclic structure (conjugated heterocyclic ring) is the ligand atom coordinating to M¹ and M². The hetero atom may be, for example, nitrogen, oxygen, sulfur or phosphorus.

BL is preferably a tetradentate ligand. And it is more preferably anionic. BL may or may not have a substituent on the cyclic structure (conjugated heterocyclic ring).

BL may be the ligand represented by the following formula (BL-A).

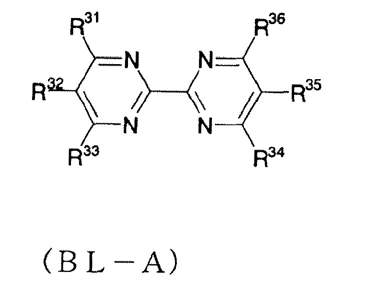

In the above formula, R³¹, R³² and R³³ independently represent hydrogen, or substituted or unsubstituted hydrocarbon, or alternatively, two or more of these together with the carbon atoms to which they are bound may form a substituted or unsubstituted aromatic hydrocarbon ring, or a substituted or unsubstituted aliphatic hydrocarbon ring. R³⁴, R³⁵ and R³⁶ independently represent hydrogen, or substituted or unsubstituted hydrocarbon, or alternatively, two or more of these together with the carbon atoms to which they are bound may form a substituted or unsubstituted aromatic hydrocarbon ring, or a substituted or unsubstituted aliphatic hydrocarbon ring.

R³¹ to R³⁶ are preferably hydrogen, alkyl or alkoxy, more preferably hydrogen or alkyl. The alkyl is preferably one containing up to six carbon atoms, more preferably methyl or ethyl. The alkoxy is preferably one containing up to six carbon atoms, more preferably methoxy or ethoxy.

It is also preferable that adjacent two of R³¹ to R³⁶ together with the carbon atoms to which they are bound form a six-membered aromatic hydrocarbon ring which may be substituted with a substituent. Examples of the substituent in the aromatic hydrocarbon ring include alkyl such as methyl and ethyl, and alkoxy such as methoxy and ethoxy.

R³¹ to R³⁶ are particularly preferably hydrogen or methyl, and R³¹ to R³⁶ are further preferably hydrogen.

BL may be the ligand represented by the following formula (BL-B).

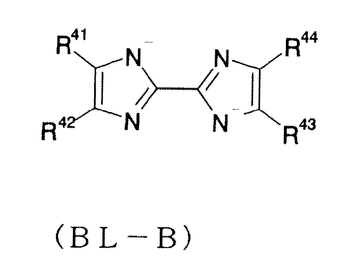

In the above formula, R⁴¹ and R⁴² independently represent hydrogen, or substituted or unsubstituted hydrocarbon, or alternatively, these together with the carbon atoms to which they are bound may form a substituted or unsubstituted aromatic hydrocarbon ring, or a substituted or unsubstituted aliphatic hydrocarbon ring. R⁴³ and R⁴⁴ independently represent hydrogen, or substituted or unsubstituted hydrocarbon, or alternatively, these together with the carbon atoms to which they are bound may form a substituted or unsubstituted aromatic hydrocarbon ring, or a substituted or unsubstituted aliphatic hydrocarbon ring.

R⁴¹ to R⁴⁴ are preferably hydrogen, alkyl or alkoxy, more preferably hydrogen or alkyl. The alkyl is preferably one containing up to six carbon atoms, more preferably methyl or ethyl. The alkoxy is preferably one containing up to six carbon atoms, more preferably methoxy or ethoxy.

It is also preferable that R⁴¹ and R⁴², and R⁴³ and R⁴⁴ together with the carbon atoms to which they are bound form a six-membered aromatic hydrocarbon ring which may be substituted with a substituent. Examples of the substituent in the aromatic hydrocarbon ring include alkyl such as methyl and ethyl, and alkoxy such as methoxy and ethoxy.

R⁴¹ to R⁴⁴ are particularly preferably hydrogen or methyl, and R⁴¹ to R⁴⁴ are further preferably hydrogen. It is also particularly preferable that R⁴¹ and R⁴² or R⁴³ and R⁴⁴ together with the carbon atoms to which they are bound form a six-membered aromatic hydrocarbon ring which may be substituted with a substituent such as methyl.

Among the ligands represented by the above formula (BL-B), the ligand represented by the following formula (BL-C) is preferable.

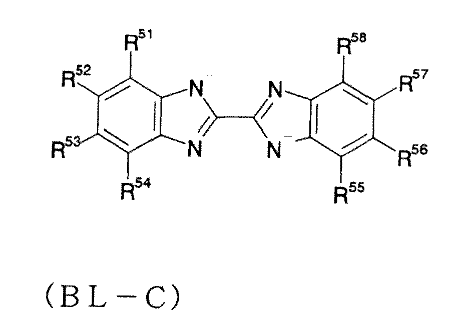

In the above formula, R⁵¹, R⁵², R⁵³ and R⁵⁴ independently represent hydrogen, or substituted or unsubstituted hydrocarbon, or alternatively, two or more of these together with the carbon atoms to which they are bound may form a substituted or unsubstituted aromatic hydrocarbon ring, or a substituted or unsubstituted aliphatic hydrocarbon ring. R⁵⁵, R⁵⁶, R⁵⁷ and R⁵⁸ independently represent hydrogen, or substituted or unsubstituted hydrocarbon, or alternatively, two or more of these together with the carbon atoms to which they are bound may form a substituted or unsubstituted aromatic hydrocarbon ring, or a substituted or unsubstituted aliphatic hydrocarbon ring.

R⁵¹ to R⁵⁸ are preferably hydrogen, alkyl or alkoxy, more preferably hydrogen or alkyl. The alkyl is preferably one containing up to six carbon atoms, more preferably methyl or ethyl. The alkoxy is preferably one containing up to six carbon atoms, more preferably methoxy or ethoxy.

It is also preferable that adjacent two of R⁵¹ to R⁵⁸ together with the carbon atoms to which they are bound form a six-membered aromatic hydrocarbon ring which may be substituted with a substituent. Examples of the substituent in the aromatic hydrocarbon ring include alkyl such as methyl and ethyl, and alkoxy such as methoxy and ethoxy.

R⁵¹ to R⁵⁸ are particularly preferably hydrogen or methyl, and R⁵¹ to R⁵⁸ are further preferably hydrogen.

Specific examples of BL include, but are not limited to, the ligands represented by the following formulas (BL-1) to (BL-4).

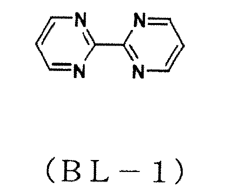

2,2'-Bipyrimidine (bpm)

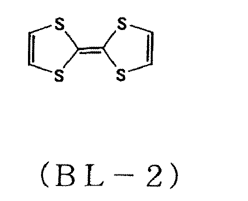

Tetrathiafluvalene (TTF)

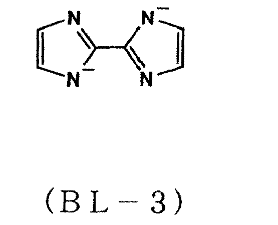

2,2'-Biimidazolato (BiIm)

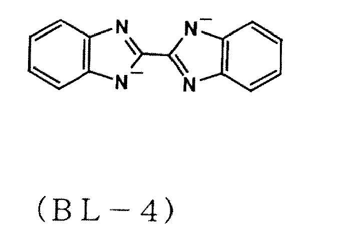

2,2'-Bibenzimidazolato (BiBzIm)
In these formulas (BL-1) to (BL-4), the heterocyclic ring and the benzene ring may be substituted with a substituent. Examples of the substituent include alkyl containing up to six carbon atoms, alkoxy containing up to six carbon atoms. Alternatively, adjacent two of the carbon atoms on the benzene ring in the formula (BL-4) may form a new benzene ring which may be substituted with a substituent.

In the case of a metal complex dye used for a photoelectric conversion element, BL is preferably the ligand represented by the above formula (BL-3) or (BL-4).

(L¹)₂M¹(BL)M²(L²)₂(X)ₙ may contain water or an organic solvent as a crystal solvent. Examples of the organic solvent include DMSO, acetonitrile, DMF, DMAC and methanol. There are no particular restrictions to the number of crystal solvents.

X is a counter ion, which is an anion when the complex [(L¹)₂M¹(BL)M²(L²)₂] is a cation, while being a cation when the complex [(L¹)₂M¹(BL)M²(L²)₂] is an anion. N is the number of counter ions needed to neutralize the charge of the complex.

When the counter ion is an anion, specific examples of X include hexafluorophosphate, perchlorate, tetraphenylborate, tetrafluoroborate, trifluoromethanesulfonate, thiocyanate, sulfate and nitrate ions, as well as halide ions such as chloride and iodide ions.

When the counter ion is a cation, specific examples of X include ammonium ion, tetrabutylammonium ion, alkali metal ions such as sodium ion, and proton.

A particularly preferable metal complex dye is the metal complex
in which L¹ is a ligand represented by the above formula (L¹-1) (including that in which H in -COOH is liberated and that in which the heterocyclic ring and the benzene ring have further a substituent); L² is a ligand represented by the above formula (L²-1) or (L²-2) (including that in which the heterocyclic ring and the benzene ring have a substituent); BL is a ligand represented by the above formula (BL-3) or (BL-4) (including that in which the heterocyclic ring and the benzene ring have a substituent); and M¹ and M² are independently selected from the group consisting of ruthenium (Ru), osmium (Os), cobalt (Co), nickel (Ni), copper (Cu) and iron (Fe).

Specific examples of an asymmetric binuclear metal complex represented by the general formula: (L¹)₂M¹(BL)M²(L²)₂(X)ₙ according to the present invention include, but are not limited to, those represented by the following formulas (D-1) to (D-16). In these formulas (D-1) to (D-16), H in -COOH may be liberated.

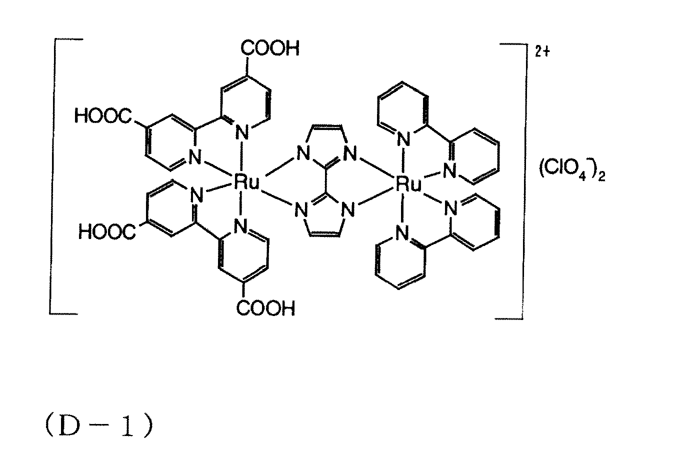

[(H₂dcbpy)₂Ru(BiIm)Ru(bpy)₂](ClO₄)₂

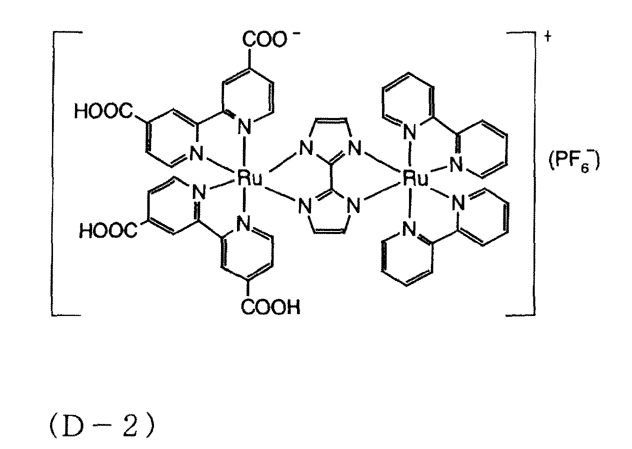

[(H₂dcbpy)(Hdcbpy)Ru(BiIm)Ru(bpy)₂](PF₆)

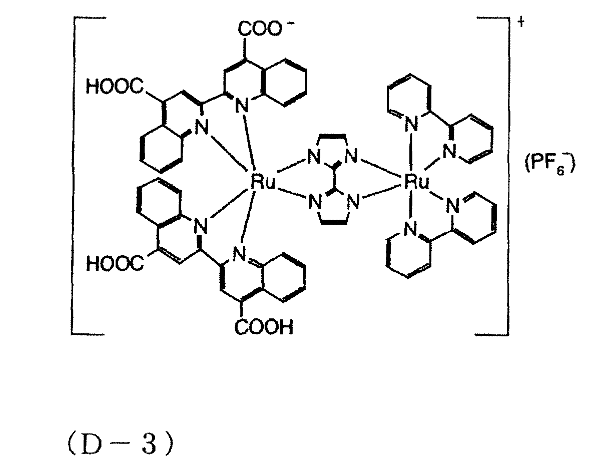

[(H₂dcbiq)(Hdcbiq)Ru(BiIm)Ru(bpy)₂](PF₆)

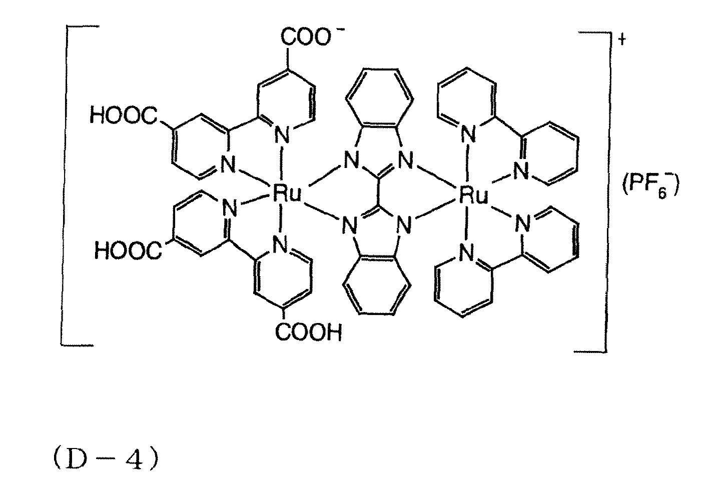

[(H₂dcbpy)(Hdcbpy)Ru(BiBzIm)Ru(bpy)₂](PF₆)

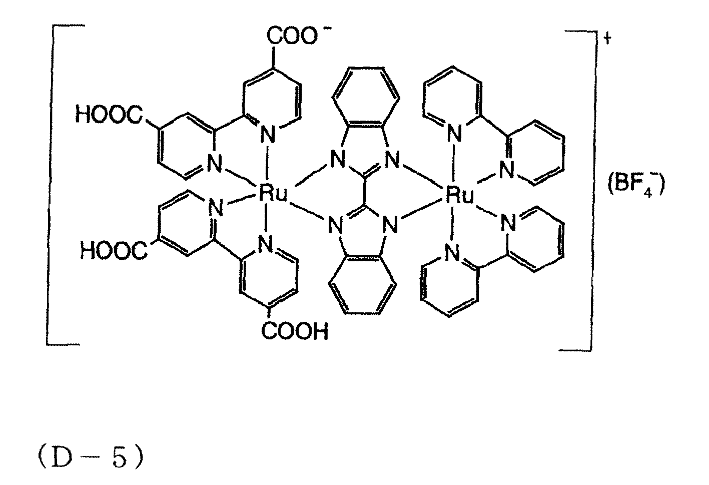

[(H₂dcbpy)(Hdcbpy)Ru(BiBzIm)Ru(bpy)₂](BF₄)

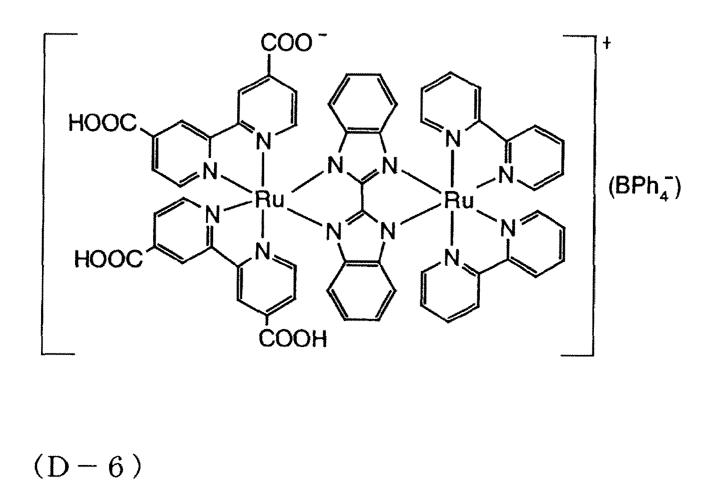

[(H₂dcbpy)(Hdcbpy)Ru(BiBzIm)Ru(bpy)₂](BPh₄)

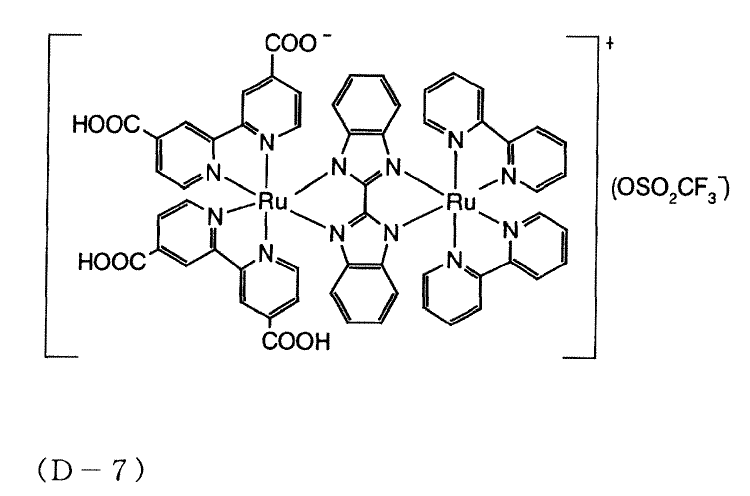

[(H₂dcbpy)(Hdcbpy)Ru(BiBzIm)Ru(bpy)₂](OSO₂CF₃)

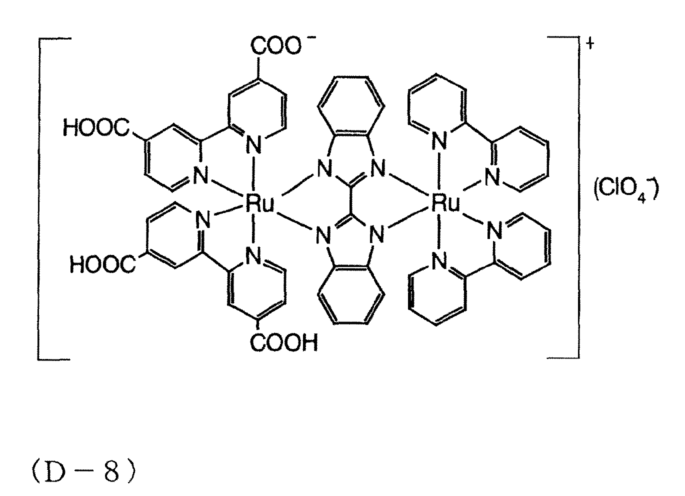

[(H₂dcbpy)(Hdcbpy)Ru(BiBzIm)Ru(bpy)₂](ClO₄)

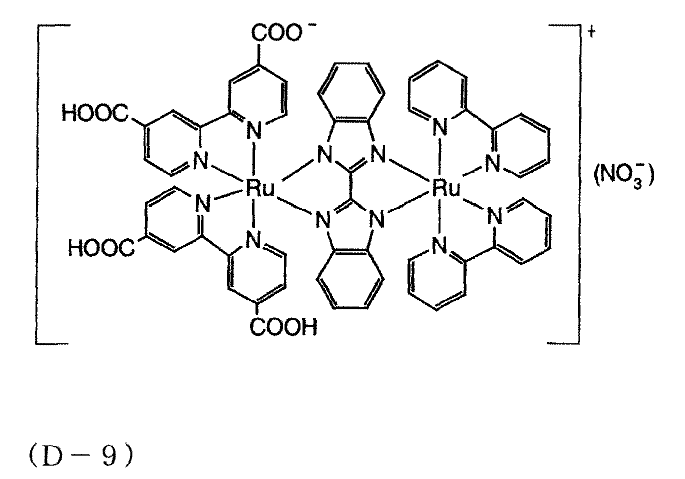

[(H₂dcbpy)(Hdcbpy)Ru(BiBzIm)Ru(bpy)₂](NO₃)

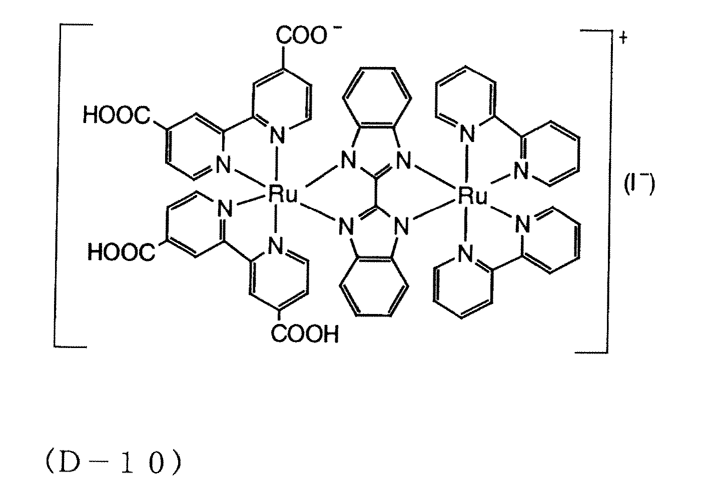

[(H₂dcbpy)(Hdcbpy)Ru(BiBzIm)Ru(bpy)₂](I)

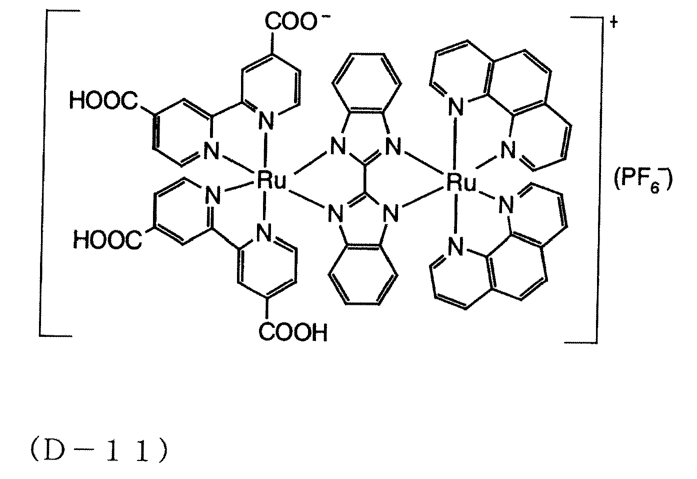

[(H₂dcbpy)(Hdcbpy)Ru(BiBzIm)Ru(phen)₂](PF₆)

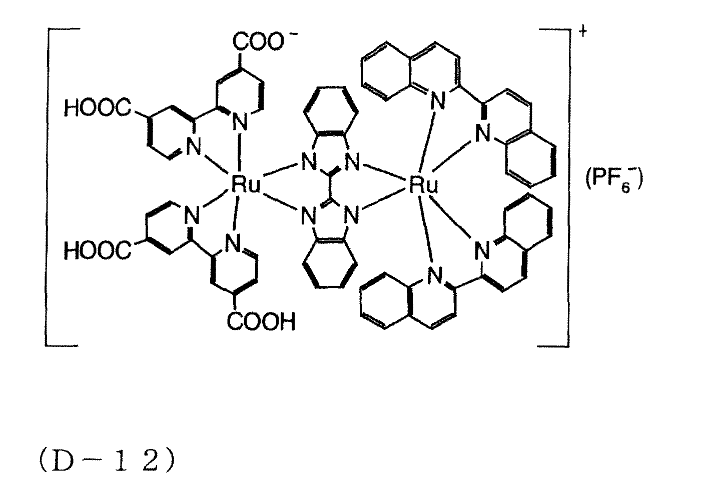

[(H₂dcbpy)(Hdcbpy)Ru(BiBzIm)Ru(biq)₂](PF₆)

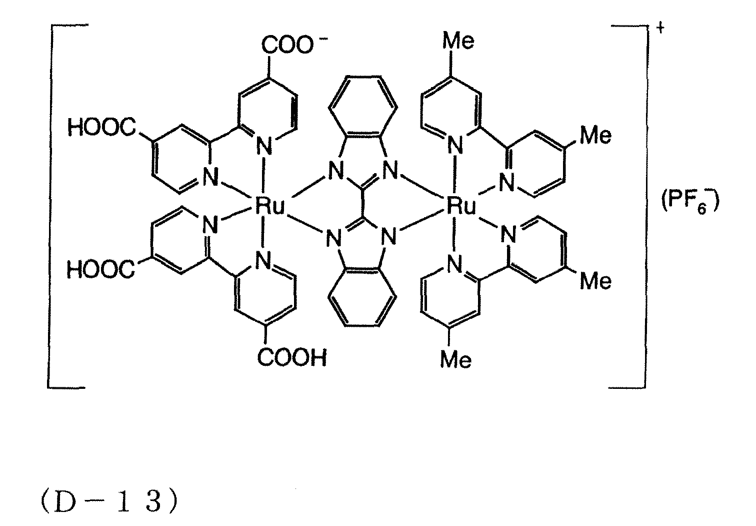

[(H₂dcbpy)(Hdcbpy)Ru(BiBzIm)Ru(dmbpy)₂](PF₆)

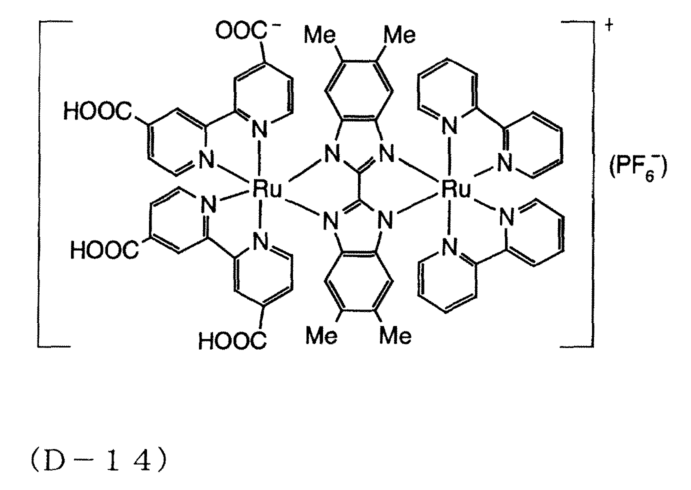

[(H₂dcbpy)(Hdcbpy)Ru(TMBiBzIm)Ru(bpy)₂](PF₆)

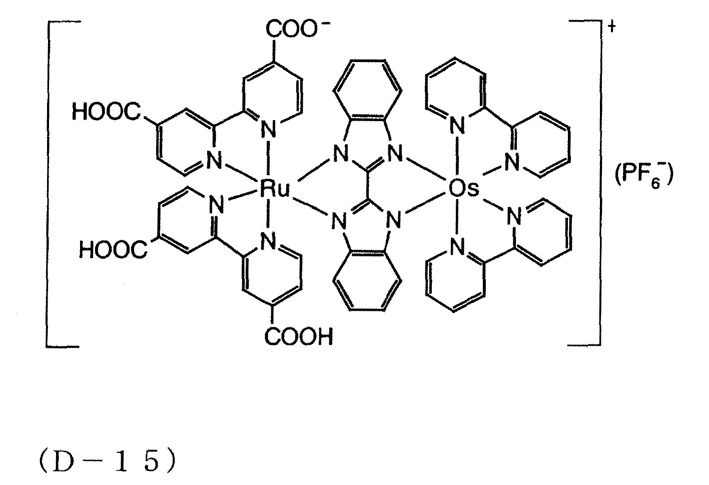

[(H₂dcbpy)(Hdcbpy)Ru(BiBzIm)Os(bpy)₂](PF₆)

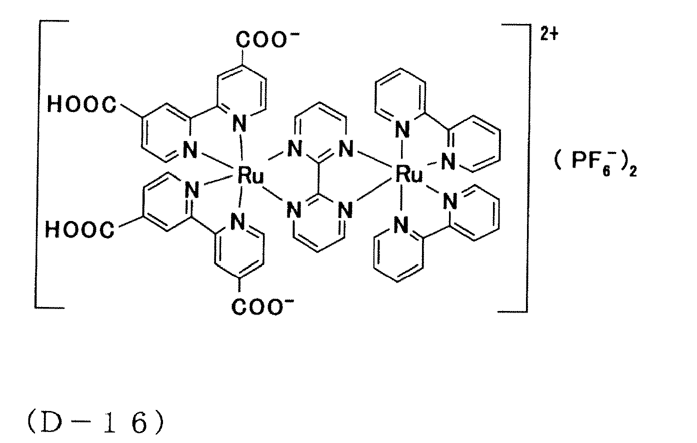

[(Hdcbpy)₂Ru(bpm)Ru(bpy)₂](PF₆)₂
The metal complex as described above may be used as a metal complex dye, and semiconductor particles sensitized by the metal complex dye may be used to produce a photochemical battery.

A photoelectric conversion element according to the present invention comprises semiconductor particles sensitized by the metal complex dye as described above. More specifically, semiconductor particles sensitized by the metal complex dye as described above are fixed on an electrode.

A conductive electrode is preferably a transparent electrode formed on a transparent substrate. Examples of a conducting agent include metals such as gold, silver, copper, platinum and palladium; indium oxide-based compounds, typified by tin-doped indium oxide (ITO); tin oxide-based compounds, typified by fluorine-doped tin oxide (FTO); and zinc oxide-based compounds.

Examples of a semiconductor particle include titanium oxide, zinc oxide, tin oxide and the like. The other examples may include indium oxide; niobium oxide; tungsten oxide; vanadium oxide; composite oxide semiconductors such as strontium titanate, calcium titanate, barium titanate and potassium niobate; cadmium or bismuth sulfide; cadmium selenide or telluride; and gallium phosphide or arsenide. The semiconductor particles may be preferably an oxide, particularly preferably titanium oxide, zinc oxide or tin oxide or a mixture comprising at least one of these.

A primary particle size of the semiconductor particles is not limited, but is generally 1 to 5,000 nm, preferably 2 to 500 nm, particularly preferably 5 to 300 nm.

A photochemical battery according to the present invention has the photoelectric conversion element as described above. More specifically, it has the photoelectric conversion element of the invention as described above, and a counter electrode as electrodes; and an electrolyte layer between them. At least one of the electrodes, i.e. the electrode used in the photoelectric conversion element of the invention and the counter electrode, is a transparent electrode.

The counter electrode acts as a cathode when it is combined with the photoelectric conversion element to form a photochemical battery. Although a substrate having a conductive layer may be used as a counter electrode, like the conductive electrode as described above, a substrate is not necessarily required for a counter electrode. A metal plate itself may be used as a counter electrode. Examples of a conducting agent used in the counter electrode include metals such as platinum and carbon and conductive metal oxides such as fluorine-doped tin oxide.

The electrolyte (oxidation-reduction pair) may be selected from any known materials without limitations. Examples of an electrolyte to be used include a combination of iodine and an iodide (for example, metal iodides such as lithium iodide and potassium iodide, or iodides of a quaternary ammonium compound such as tetrabutylammonium iodide, tetrapropylammonium iodide, pyridinium iodide and imidazolium iodide); a combination of bromine and a bromide; a combination of chlorine and a chloride; a combination of an alkylviologen and a reductant thereof; quinone/hydroquinone; transition metal ion pair such as iron (II)/iron (III) ions, copper (I)/copper (II) ions, manganese(II)/manganese(III) ions, and cobalt (II)/cobalt (III) ions; a combination of complex ions such as ferrocyanide/ferricyanide, cobalt (II) tetrachloride/cobalt (III) tetrachloride, cobalt (II) tetrabromide/cobalt (III) tetrabromide, iridium(II) hexachloride/iridium (III) hexachloride, ruthenium (II) hexacyanide/ruthenium (III) hexacyanide, rhodium(II) hexachloride/rhodium(III) hexachloride, rhenium (III) hexachloride/rhenium (IV) hexachloride, rhenium (IV) hexachloride/rhenium (V) hexachloride, osmium (III) hexachloride/osmium (IV) hexachloride, and osmium (IV) hexachloride/osmium (V) hexachloride; a complex formed with a transition metals such as cobalt, iron, ruthenium, manganese, nickel and rhenium, and a conjugated heterocyclic ring and derivative thereof such as bipyridine and derivative thereof, terpyridine and derivative thereof, and phenanthroline and derivative thereof; a complex of cyclopentadiene or derivative thereof and a metal such as ferrocene/ferrocenium ion, cobaltocene/cobaltocenium ion, and ruthenocene/ruthenocenium ion; and porphyrin compounds. A preferable electrolyte is a combination of iodine and lithium iodide or an iodide of a quaternary ammonium compound. The electrolyte may be a solution in an organic solvent, a molten salt, a so-called gel electrolyte in which the electrolyte is impregnated in a polymer matrix, or a solid electrolyte.

A photochemical battery according to the present invention may be produced by any of conventional processes.

For example, on a transparent electrode is applied a paste of semiconductor particle such as an oxide, which is then calcined to form a thin film of the semiconductor particles. When the semiconductor-particle thin film is titania, calcination is carried out at a temperature of 450 °C and a reaction time of 30 minutes. This transparent electrode with the thin film is immersed in a dye solution for supporting the dye on semiconductor particles, to produce a photoelectric conversion element. Then, the photoelectric conversion element is combined with a transparent electrode on which platinum or carbon has been vapor-deposited as a counter electrode, and an electrolyte solution is infiltrated into a gap between them to produce a photochemical battery according to the present invention.

### EXAMPLES

The present invention will be more specifically described with reference to the following examples. However, the present invention is not limited to these Examples.

### (Comparative Example 1)

Synthesis of the binuclear metal complex dye (D-4) [(H₂dcbpy)(Hdcbpy)Ru (BiBzIm)Ru(bpy)₂](PF₆)

### (Synthesis of D-4 isolated at pH 2.5)

### 1. Synthesis of the mononuclear metal complex (H₂dcbpy)₂RuCl₂ (M¹C-1)

Under nitrogen atmosphere, in a 500 mL three-necked flask were placed commercially available RuCl₃·3H₂O (2.53 g, 9.68 mmol), H₂dcbpy (4.50 g, 18.4 mmol) and 300 mL of N,N-dimethylformamide, and the mixture was refluxed under irradiation with 2.45 GHz microwave for 45 minutes. After cooling down, the mixture was filtered, and the resulting filtrate was evaporated under vacuum to dryness. The resulting residue was washed with acetone/diethyl ether (1:4), 300 mL of 2 mol/L hydrochloric acid was added thereto, and the mixture was stirred with ultrasonic for 20 minutes and then stirred for 2 hours without ultrasonic. After stirring, an insoluble material was collected by filtration and washed with 2 mol/L hydrochloric acid, acetone/diethyl ether (1:4) and diethyl ether. After drying under vacuum, 5.75 g of M¹C-1 was obtained.

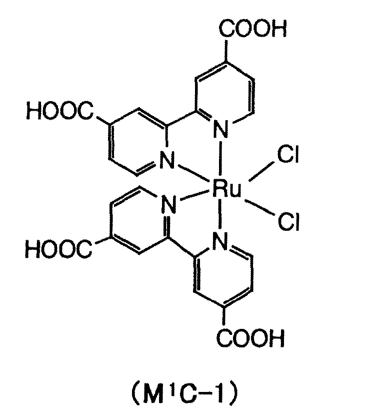

### 2. Synthesis of the mononuclear metal complex (BiBzIm)Ru(bpy)₂ (M²C-2)

Under nitrogen atmosphere, in a 300 mL three-necked flask were placed Ru(bpy)₂Cl₂ (4.02 g, 7.7 mmol), 2,2'-bibenzimidazole (BiBzImH₂) (2.18 g, 9.3 mmol) prepared as described in Inorg. Chem., 34, 5979 (1995) and 100 mL of ethylene glycol, and the mixture was refluxed under irradiation with 2.45 GHz microwave for 5 minutes. After cooling down, 35 mL of 10 % solution of lithium methoxide in methanol was added to the mixture, and then the mixture was irradiated with 2.45 GHz microwave at 60 °C for 10 minutes. After cooling down, 200 mL of water was added to the mixture. The resulting mixture was stirred, and then the precipitate was collected by filtration. The precipitate was washed with water, cold methanol and diethyl ether. After drying under vacuum, 5.7708 g of M²C-2 was obtained. Subsequently, under nitrogen atmosphere, 5.77 g of the resulting precipitate was added to 200 mL of methanol, and then 10 mL of 10 % solution of lithium methoxide in methanol was added thereto. The suspension was refluxed for 1 hour. After cooling down, the precipitate was collected by filtration and washed with cold methanol, water and diethyl ether. After drying under vacuum, 5.02 g of M²C-2 was obtained.

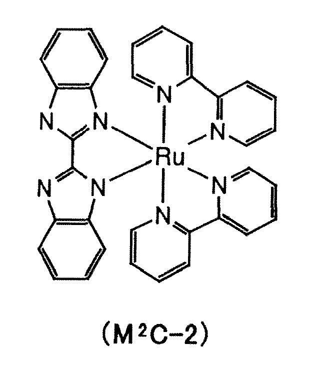

### 3. Synthesis of D-4

Under nitrogen atmosphere, in a 300 mL three-necked flask were placed M¹C-1 (0.601 g, 0.86 mmol) and 100 mL of ethanol/water (1:1), and then 3.7 mL of a 1 mol/L aqueous sodium hydroxide solution was added dropwise thereto to give a solution. To the solution was added M²C-2 (0.621 g, 0.91 mmol), and the mixture was refluxed under irradiation with 2.45 GHz microwave for 30 minutes. After the mixture cooled down, a small amount of the insoluble material was removed by filtration, and ethanol in the filtrate was evaporated under vacuum. The resulting suspension was filtered, and a 0.5 mol/L aqueous hexafluorophosphoric acid solution was added dropwise to the filtrate until the pH became 2.5. The resulting mixture was cooled at 4 °C overnight. And then, the precipitated complex was collected by filtration, and washed with an aqueous hexafluorophosphoric acid solution at pH 2.5, acetone/diethyl ether (4:1) and diethyl ether. After drying under vacuum, 1.11 g of D-4 was obtained.

### (Example 1)

### (Synthesis of D-4 isolated at pH 2.8)

Under nitrogen atmosphere, in a 500 mL three-necked flask were placed M¹C-1 (1.50 g, 2.16 mmol) and 300 mL of ethanol/water (1:1), and then 8.7 mL of a 1 mol/L aqueous sodium hydroxide solution was added dropwise thereto to give a solution. To the solution was added M²C-2 (1.55 g, 2.27 mmol), and the mixture was refluxed under irradiation with 2.45 GHz microwave for 30 minutes. After the mixture cooled down, a small amount of the insoluble material was removed by filtration, and ethanol in the filtrate was evaporated under vacuum. The resulting suspension was filtered, and a 0.5 mol/L aqueous hexafluorophosphoric acid solution was added dropwise to the filtrate until the pH became 2.8. The resulting mixture was cooled at 4 °C overnight. And then, the precipitated complex was collected by filtration, and washed with an aqueous hexafluorophosphoric acid solution at pH 2.8, acetone/diethyl ether (4:1) and diethyl ether. After drying under vacuum, 2.61 g of D-4 isolated at pH 2.8 was obtained.

### (Example 2)

### (Synthesis of D-4 isolated at pH 3.5)

1.46 g of D-4 was obtained as described in "Synthesis of D-4". D-4 (1.13 g) thus obtained was suspended in water, and then an aqueous sodium hydroxide solution was added dropwise to the suspension until the pH became 10, to redissolve D-4 in water. Subsequently, a 0.5 mol/L aqueous hexafluorophosphoric acid solution was added dropwise to the resulting solution until the pH became 3.5. The resulting mixture was cooled at 4 °C overnight. And then, the precipitated complex was collected by filtration, and washed with an aqueous hexafluorophosphoric acid solution at pH 3.5, acetone/diethyl ether (4:1) and diethyl ether. After drying under vacuum, 0.866 g of D-4 isolated at pH 3.5 was obtained.

### (Example 3)

### (Synthesis of D-4 isolated at pH 3.8)

1.21 g of D-4 was obtained as described in "Synthesis of D-4". D-4 (1.02 g) thus obtained was suspended in water, and then an aqueous sodium hydroxide solution was added dropwise to the suspension until the pH became 10, to redissolve D-4 in water. Subsequently, a 0.5 mol/L aqueous hexafluorophosphoric acid solution was added dropwise to the resulting solution until the pH became 3.8. The resulting mixture was cooled at 4 °C overnight. And then, the precipitated complex was collected by filtration, and washed with acetone/diethyl ether (4:1) and diethyl ether. After drying under vacuum, 0.681 g of D-4 isolated at pH 3.8 was obtained.

### (Example 4)

### (Synthesis of D-4 isolated at pH 4.0)

1.15 g of D-4 was obtained as described in "Synthesis of D-4". D-4 (1.12 g) thus obtained was suspended in water, and then an aqueous sodium hydroxide solution was added dropwise to the suspension until the pH became 10, to redissolve D-4 in water. Subsequently, a 0.02 mol/L aqueous hexafluorophosphoric acid solution was added dropwise to the resulting solution until the pH became 4.0. The resulting mixture was cooled at -20 °C overnight. And then, the precipitated complex was collected by filtration, and washed with acetone/diethyl ether (4:1) and diethyl ether. After drying under vacuum, 0.353 g of D-4 isolated at pH 4.0 was obtained.

### (Example 5)

### 1. Preparation of a porous titania electrode

### (Preparation of a porous titania electrode)

A titania paste PST-18NR for a transparent layer and a titania paste PST-400C for a diffusion layer (made by Catalysts & Chemicals Industries Co., Ltd. ) were applied onto a transparent conductive glass electrode (made by Asahi Glass Co., Ltd.) using a screen printer. The film thus obtained was aged in an atmosphere at 25 °C and 60 % for 5 minutes, and then the aged film was calcined at 450 °C for 30 minutes. After cooling down, the film was again subjected to the same process to achieve the predetermined thickness, thereby preparing a 16 mm² porous titania electrode.

### 2. Preparation of a dye-adsorbed porous titania electrode

A porous titania electrode was immersed in a saturated dye solution of D-4 in IPA at 30 °C for 20 hours, and then it was dried to prepare a dye-adsorbed porous titania electrode for determination of ordinary photoelectric conversion efficiency. Another porous titania electrode was immersed in a saturated dye solution of D-4 in t-butanol/acetonitrile (1:1) at 30 °C for 40 hours, and then it was dried to prepare a dye-adsorbed porous titania electrode for determination of durability.

### 3. Preparation of a photochemical battery

A dye-adsorbed porous titania electrode thus obtained was combined with a platinum plate (counter electrode). Then, an electrolyte solution was infiltrated into a gap between these electrodes by the capillary action to prepare a photochemical battery. The electrolyte solution used for the photochemical battery for determination of ordinary photoelectric conversion efficiency was a solution obtained by dissolving lithium iodide, iodine, 4-t-butylpyridine and 1,2-dimethyl-3-propylimidazolium iodide in 3-methoxypropionitrile to 0.1, 0.05, 0.5 and 0.6 mol/L, respectively. The electrolyte solution used for the photochemical battery for determination of durability was a solution obtained by dissolving iodine, methylbenzimidazole and 1,2-dimethyl-3-propylimidazolium iodide in γ-butyrolactone to 0.1, 0.5 and 0.6 mol/L, respectively.

### 4. Determination of photoelectric conversion efficiency

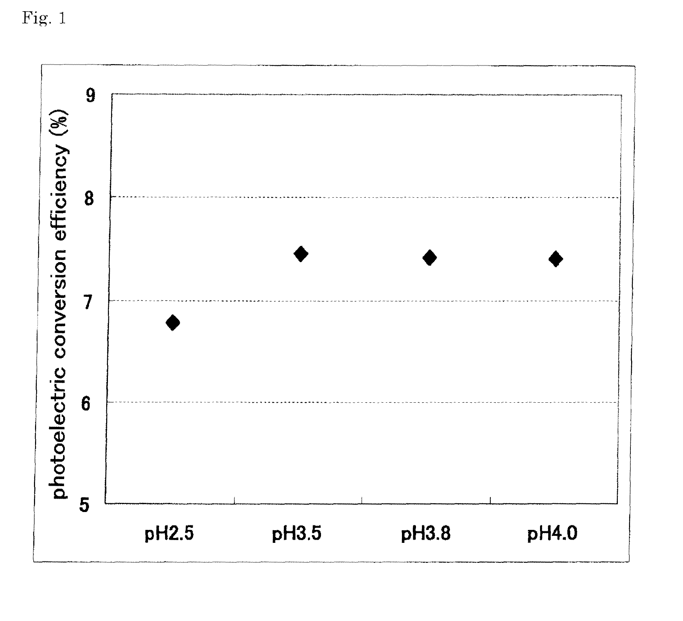

The photoelectric conversion efficiency of the photochemical battery thus prepared was determined under irradiation with artificial solar light at 100 mW/cm² using a solar simulator (made by EKO Instruments Co., Ltd.). The measurement result on the photoelectric conversion efficiency is shown in FIG. 1.

As seen from FIG. 1, the photochemical batteries comprising the binuclear metal complex dyes of the present invention isolated at a pH of 3.5 or higher had higher photoelectric conversion efficiency than the photochemical battery comprising the binuclear metal complex dye isolated at pH 2.5.

### 5. Determination of durability

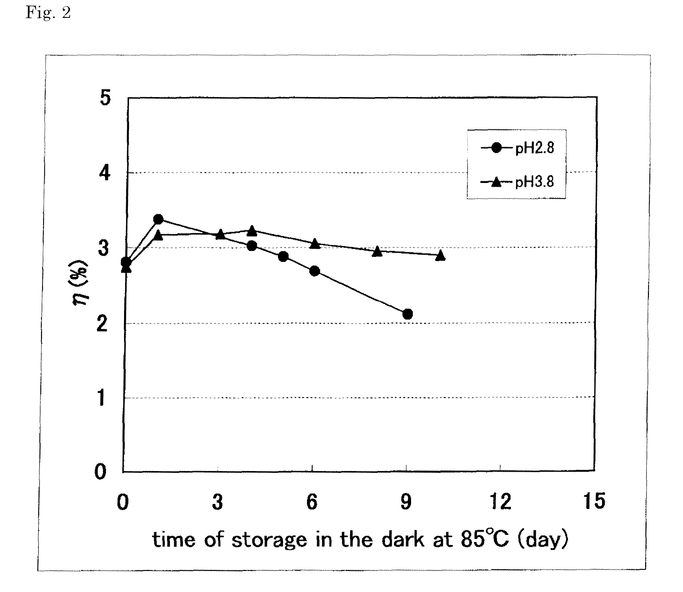

The photochemical battery thus prepared was left in the dark at 85 °C for a predetermined time. After the photochemical battery was returned to room temperature, the photoelectric conversion efficiency (η) was determined under irradiation with artificial solar light at 100 mW/cm² using a solar simulator (made by EKO Instruments Co., Ltd.). The change in the photoelectric conversion efficiency with respect to the time period during which the photochemical battery was left in the dark at 85 °C is shown in FIG. 2.

As seen from FIG. 2, the photochemical battery comprising the binuclear metal complex dye isolated at pH 3.8 had higher durability than the photochemical battery comprising the binuclear metal complex dye isolated at pH 2.8.

### Industrial Applicability

As described above, according to the present invention, there may be provided a metal complex dye to produce a photochemical battery having higher photoelectric conversion efficiency and higher durability.

## Claims

1. A process for production of an asymmetric binuclear metal complex, comprising a step of:
isolating the binuclear metal complex by adjusting the pH of the solution containing the binuclear metal complex to a value higher than 2.5;
wherein
the binuclear metal complex is a complex represented by the general formula: (L¹)₂M¹(BL)M²(L²)₂(X)ₙ
in which
M¹ and M², which may be the same as or different from each other, represent a transition metal;
L¹ and L², which are different from each other, represent a chelate ligand capable of multidentate coordination, and two L¹s may be different from each other, and two L²s may be different from each other;
BL represents a bridging ligand having at least two cyclic structures each containing a hetero atom, the hetero atoms contained in the cyclic structures being ligand atoms coordinating to M¹ and M²;
X represents a counter ion; and
n is the number of counter ions needed to neutralize the charge of the complex.

2. The production process as claimed in Claim 1, wherein the binuclear metal complex represented by the formula: (L¹)₂M¹(BL)M²(L²)₂(X)ₙ is isolated by adjusting the pH of the solution to 2.7 to 5.

3. The production process as claimed in Claim 2, wherein the binuclear metal complex represented by the formula: (L¹)₂M¹(BL)M²(L²)₂(X)ₙ is isolated by adjusting the pH of the solution to 3.3 to 5.

4. The production process as claimed in any of Claims 1 to 3,
wherein the solution is a reaction solution obtained by reacting a mononuclear metal complex represented by the formula: ((L¹)₂M¹Cl₂ wherein M¹ and L¹ have the meanings indicated above) with a mononuclear metal complex represented by the formula: (BL)M²(L²)₂ (wherein M², L² and BL have the meanings indicated above) in the presence of a base in a solvent, or a solution obtained by dissolving a binuclear metal complex represented by the formula: (L¹)₂M¹(BL)M²(L²)₂(X)ₙ in water.

5. An asymmetric binuclear metal complex produced by the process as claimed in any of Claims 1 to 4, and represented by the general formula: (L¹)₂M¹(BL)M²(L²)₂(X)ₙ
in which
M¹ and M², which may be the same as or different from each other, represent a transition metal;
L¹ and L², which are different from each other, represent a chelate ligand capable of multidentate coordination, and two L¹s may be different from each other, and two L²s may be different from each other;
BL represents a bridging ligand having at least two cyclic structures each containing a hetero atom, the hetero atoms contained in the cyclic structures being ligand atoms coordinating to M¹ and M²;
X represents a counter ion; and
n is the number of counter ions needed to neutralize the charge of the complex.

6. A metal complex dye comprising an asymmetric binuclear metal complex produced by the process as claimed in any of Claims 1 to 4, and represented by the general formula: (L¹)₂M¹(BL)M²(L²)₂(X)ₙ
in which
M¹ and M², which may be the same as or different from each other, represent a transition metal;
L¹ and L², which are different from each other, represent a chelate ligand capable of multidentate coordination, and two L¹s may be different from each other, and two L²s may be different from each other;
X represents a counter ion;
n is the number of counter ions needed to neutralize the charge of the complex;
BL represents a bridging ligand having at least two cyclic structures each containing a hetero atom, the hetero atoms contained in the cyclic structures being ligand atoms coordinating to M¹ and M²; and
L¹ contains a substituent capable of attaching to a semiconductor particle; and
LUMOs are predominantly distributed in (L¹)₂M¹.

7. A photoelectric conversion element comprising semiconductor particles sensitized by the metal complex dye as claimed in Claim 6.

8. The photoelectric conversion element as claimed in Claim 7,
wherein the semiconductor particle is selected from the group consisting of titanium oxide, zinc oxide and tin oxide.

9. A photochemical battery comprising the photoelectric conversion element as claimed in any of Claims 7 to 8.
